# EUROPEAN PATENT APPLICATION

(11) **EP 4 767 921 A1**
(43) Date of publication of application: **01.07.2026**
(21) Application number: 25202469.0
(22) Date of filing: 16.09.2025
(51) Int. Cl.: A61B 1/008, A61B 1/005

(54) **ENDOSCOPIC ARTICULATING TUBE**

(30) Priority: 26.12.2024 KR 20240197285
(71) Applicant: Dyne Medical Group Inc., Gimpo-si, Gyeonggi-do 10071 (KR)
(72) Inventor: LEE, Sung Hoon, 21982 Yeonsu-gu, Incheon (KR); HWANG, Eun Soon, 10077 Gimpo-si, Gyeonggi-do (KR)
(74) Representative: Berggren Oy

(57) **Abstract**

Disclosed is an endoscopic articulating tube comprising a plurality of tubular unit segments that are articulately connected in series, allowing an endoscopic insertion unit to perform a flexion and extension motion by pulling and releasing a wire under control of a separate operating unit.

## Description

### BACKGROUND

### Technical Field

The present disclosure relates to an endoscopic articulating tube, and more particularly, to an endoscopic articulating tube configured to bend and extend under control of an endoscopic operating unit.

### Description of Related Technology

In general, an endoscope is a general term for a medical device used to examine the inside of the human body for medical purposes, and a fiber endoscope is a very flexible and easily operable device and is a semi-solid device capable of examining even the digestive tract, which was previously unobservable. The fiber endoscope is formed of a bundle of thin glass fibers and capable of being easily bent and twisted, and since strong light is projected forward and backward to every corner, an examiner is able to examine the inside clearly.

Recently, such endoscopes have been equipped with procedural or surgical instruments for minimally invasive procedures, and have been used for collecting samples of cells or tissues, excising and removing polyps or tumors, and performing other procedures or surgeries.

The endoscopic instruments include an esophagoscope, which is a rigid tube used to examine the esophagus; a bronchoscope, which is used to examine the bronchus; a gastroscope, which is a flexible, lighted tube used to examine the stomach; a proctoscope, which is a rigid tube used to examine the rectum or descending colon; and a cystoscope, which is a lighted tube used to examine the bladder through the urethra. Other instruments include a laparoscope, a thoracoscope, a cardioscope, a mediastinoscope, and similar instruments.

Recently, an articulating structure for an endoscopic insertion unit has been proposed to locally change the direction of an end effector disposed at a distal end of the endoscopic insertion unit. In general, the articulating structure performs pan and/or tilt steering of a tip using a plurality of wires passing through the articulating structure.

As an example of the articulating structure, there is a device in which a plurality of segments are connected in series, and steering is performed by relatively moving adjacent segments.

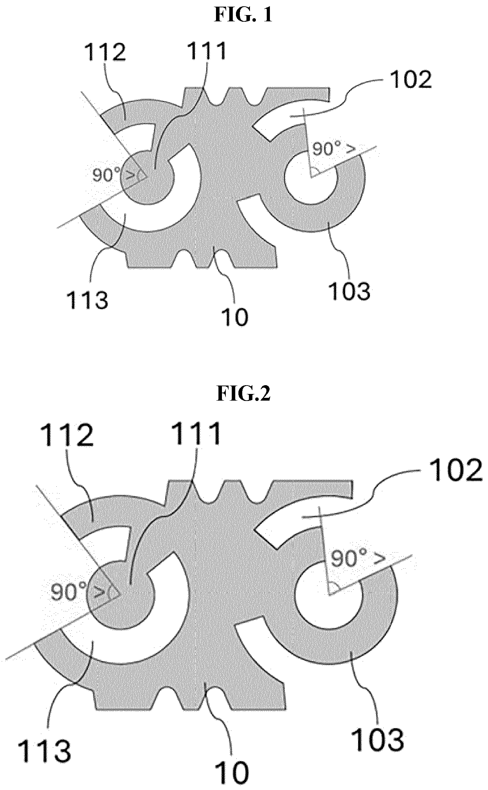

As shown in FIGS. 1 and 2, the conventional articulating structure for an endoscopic insertion unit as described above includes: a plurality of segments 10; a first C-shaped coupling portion 101 formed at one end of each of the segments 10 in a direction forming a first acute angle (less than 90 degrees) with an axial direction of the segments 10; a first C-shaped notch 102 provided to surround an outer side of the first C-shaped coupling portion 101; a second C-shaped coupling portion 111 provided at the other end of each of the segments 10 to be put-in to the first C-shaped notch 102 of another segment 10; a second C-shaped notch 112 and a spherical protrusion 113, which are configured to be put-in to the first C-shaped coupling portion 101 of another segment 10, are provided on an inner side of the second C-shaped coupling portion 111. The second C-shaped notch 112 is configured to surround the spherical protrusion 113. Thus, when two adjacent segments 10 are connected in series, the spherical protrusion 113 of a first segment 10 is put-in to the inside of the first C-shaped coupling portion 101 of a second segment 10, whereby the first C-shaped coupling portion 101 of the second segment 10 is coupled to the second C-shaped notch 112 of the first segment 10, and simultaneously, the second C-shaped coupling portion 111 of the first segment 10 is coupled to the first C-shaped notch 102 of the second segment 10.

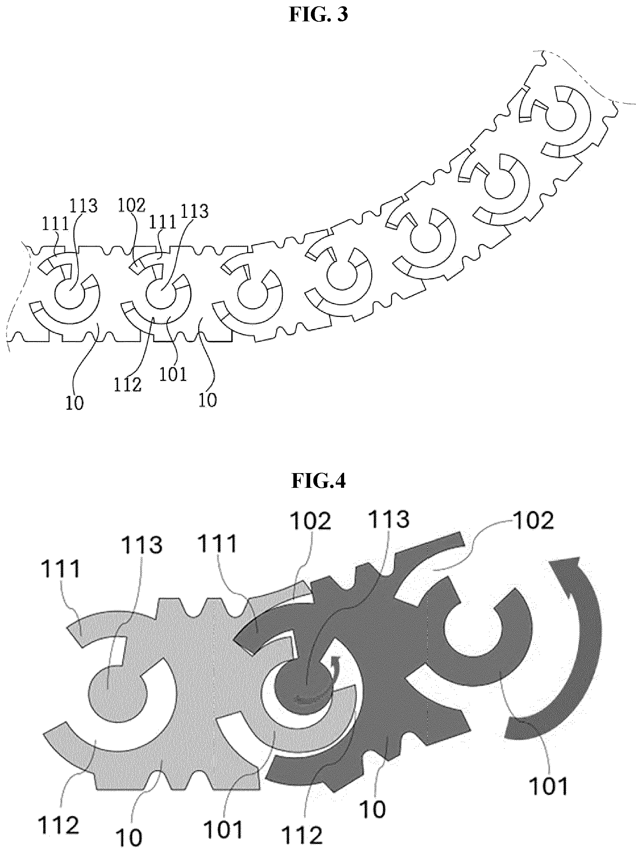

In the conventional articulating structure for an endoscope as described, the first C-shaped coupling portion 101 of the first segment 10 is fitted into the second C-shaped notch 112 of the second segment 10 and rotatable at a predetermined angle while surrounds the spherical protrusion 113 , and the second C-shaped coupling portion 111 of the second segment 10 is fitted into the first C-shaped notch 102 of the first segment 10 and rotatable at a predetermined angle while surrounding the first C-shaped coupling portion 101, and thereby, as illustrated in FIG. 3, the direction of an end effector disposed at a distal end of the endoscopic insertion unit may be locally changed.

The conventional articulating structure for an endoscope is a simple structure in which two coupling portions surround and engage with two notch portions and the spherical projection 113, as described above, and thus, there is a problem in that a bonding force between different adjacent segments 10 is weak, and accordingly, a put-in portion is easily disengaged, which serves as a critical problem in an endoscope used for insertion into a human body. In particular, as shown in FIG. 4, when a pair of left and right wires configured to be pulled or released by an operating unit for a flexion and extension motion of the articulating structure are forcibly pulled, a put-in portion formed among the two coupling portions, the two notch portions, and the spherical protrusion may deviate beyond an allowable rotation angle and become disengaged.

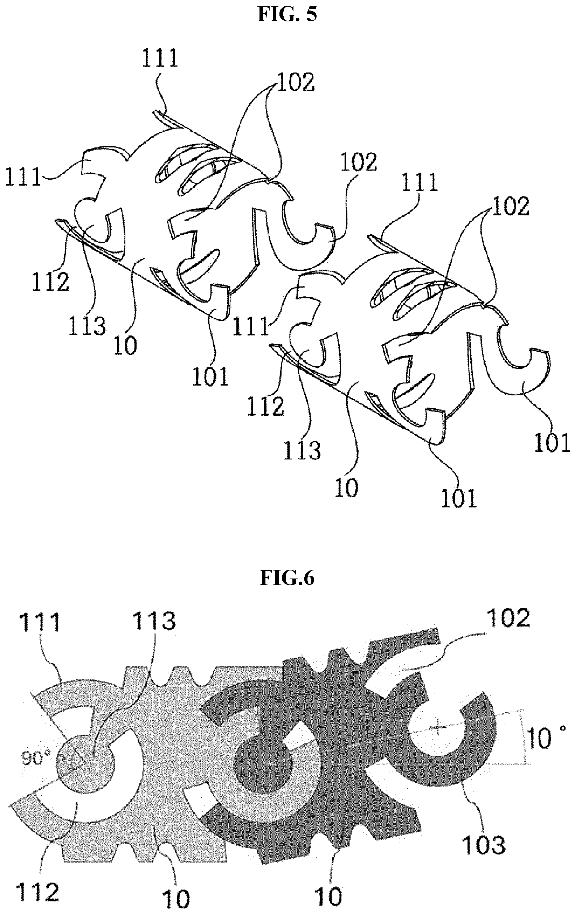

In particular, as illustrated in FIG. 5, the first C-shaped coupling portion 101 and the first C-shaped notch 102 are symmetrically formed on both facing sides of one end of each segment 10, and the second C-shaped coupling portion 111, the second C-shaped notch 112, and the spherical protrusion 113 are symmetrically formed on both facing sides of the other end. Therefore, as described above, when a pair of left and right wires configured to be pulled or released by the operating unit for a flexion and extension motion of the articulating structure are forcibly pulled, the problem is aggravated in that a put-in portion formed among the two coupling portions, the two notch portions, and the spherical projection deviates beyond an allowable rotation angle and becomes disengaged.

In addition, in the case of the conventional articulating structure for an endoscope as described above, since the openings of the first C-shaped coupling portion 101 and the second C-shaped coupling portion 111 are each formed at a narrow angle (an angle less than 90 degrees), as illustrated in FIG. 6, the rotation angle at each put-in portion is extremely limited. As a result, the articulation bending angle of the articulating structure becomes inevitably narrow. Thus, when viewed as a whole, the articulating structure bends into a large curve, creating restrictions when procedures or surgeries are performed in narrow regions of the human body.

For reference, although there are some overlapping reference numerals between the drawings of the related art and those of the present disclosure, such reference numerals are added to facilitate understanding of each technology, and therefore, it is desirable to understand and interpret them separately.

### [Related Art Literature]

### [Patent Document]

Japanese Registered Patent Publication No. 6902170 (Published on December 17, 2020)

### SUMMARY

An object of the present disclosure is to provide an endoscopic articulating tube that ensures a robust put-in structure for an articulating portion in which continuously arranged segments are connected to perform a flexion and extension motion, and that secures a maximum articulation angle in a distal direction of an endoscopic insertion unit, particularly where an end effector is located, so as to minimize a bending radius.

In one aspect of the present disclosure, there is provided an endoscopic articulating tube including a plurality of tubular unit segments that are articulately connected in series, allowing an endoscopic insertion unit to perform a flexion and extension motion by pulling and releasing a wire under control of a separate operating unit. The endoscopic articulating tube includes: a first coupling unit including a plurality of coupling parts concentrically arranged around a spherical block portion protruding from one side connecting end of each of the segments, each coupling part including a cape block portion and a bay space portion disposed at different radii and respectively forming an acute angle and an obtuse angle; and a second coupling unit including a plurality of coupling parts concentrically arranged around a spherical space portion provided at the other side connecting end of each of the segments, each coupling part including a bay space portion and a cape block portion disposed at different radii and respectively forming an obtuse angle and an acute angle. The spherical block portion, the cape block portion, and the bay space portion of the first coupling unit are put-in and articulately coupled to the spherical space portion, the bay space portion, and the cape block portion of the second coupling unit of an adjacent segment.

The first coupling unit may be provided on a first side in a first direction, which corresponds to one side connecting end of each of the segments, and the second coupling unit is provided on a second side in the first direction. The second coupling unit may be provided on a first side in a second direction, which corresponds to the other side connecting end of each of the segments, and the first coupling unit is provided on a second side in the same second direction.

The first coupling unit may be formed of a first-first coupling part, a first-second coupling part, and a first-third coupling part around three concentric circles having different radii centered around a spherical block portion.

The first-first coupling part may include a plurality of first-first cape block portions and first-first bay space portions alternately arranged around a first concentric circle surrounding the spherical block portion. The first-second coupling part may include a plurality of first-second cape block portions and first-second bay space portions alternately arranged around a second concentric circle surrounding the first-first coupling part. The first-third coupling part may include a plurality of first-third cape block portions and first-third bay space portions alternately arranged around a third concentric circle surrounding the first-second coupling part.

The first-first cape block portions, the first-second bay space portions, and the first-third cape block portions may be partially alternately formed in a radial direction of the concentric circles, and the first-first bay space portions, the first-second cape block portions, and the first-third bay space portions may be partially alternately formed in the radial direction of the concentric circles.

The second coupling unit may be formed of a second-first coupling part, a second-second coupling part, and a second-third coupling part disposed around three concentric circles having different radii centered around a spherical space portion.

The second-first coupling part may include a plurality of second-first cape block portions and second-first bay space portions alternately arranged around the first concentric circle surrounding the spherical space. The second-second coupling part may include a plurality of second-second cape block portions and second-second bay space portions alternately arranged around a second concentric circle surrounding the second-first coupling part. The second-third coupling part may include a plurality of second-third cape block portions and second-third bay space portions alternately arranged around a third concentric circle surrounding the second-second coupling part.

The second-first cape block portions, the second-second bay space portions, and the second-third cape block portions may be partially alternately formed in the radial direction of the concentric circles. The second-first bay space portions, the second-second cape block portions, and the second-third bay space portions may be partially alternately formed in the radial direction of the concentric circles.

When two adjacent segments are connected in a straight line direction, the spherical block portion of the first coupling unit may be coupled to the spherical space portion of the second coupling unit, and each of the cape block portions and bay space portions forming the first-first coupling part, the first-second coupling part, and the first-third coupling part of the first coupling unit may be coupled to a corresponding one of the cape block portions and bay space portions forming the second-first coupling part, the second-second coupling part, and the second-third coupling part of the second coupling unit, so that each of the cape block portions is rotatably movable within a corresponding one of the bay space portions within a predetermined angular range.

The first and second coupling units may be provided to protrude at the one side connecting end and the other side connecting end of each of the segments, and flexion grooves may be provided on outer circumferential surface of one side connecting end and the other side connecting end, excluding areas where the first and second coupling units are provided to protrude, to secure an articulation range during bending.

Wire support portions, each formed as an inwardly recessed cutout, may be provided on upper and lower outer circumferential surfaces of each of the segments in a direction orthogonal to a direction in which the first and second coupling units are provided.

The wire support portions may be arranged in every other segment among continuously connected segments, thereby being arranged in a stepping-stone pattern.

The segments continuously arranged in a straight direction to form the articulating tube may be arranged such that a length of a tubular body gradually becomes shorter toward a distal end where the end effector is located.

### BRIEF DESCRIPTION OF THE DRAWINGS

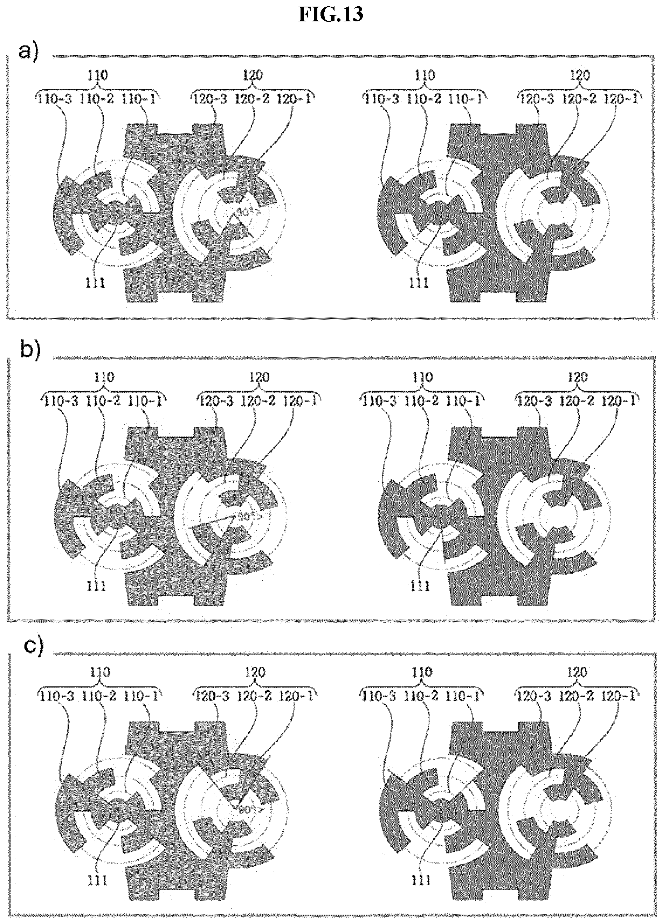

FIG. 1 is a perspective view illustrating a portion of a conventional endoscopic articulating tube.
FIG. 2 is a side view illustrating a unit segment included in a conventional endoscopic articulating tube.
FIG. 3 is a side view illustrating a state in which a conventional endoscopic articulating tube is bent by a flexion and extension operation of an articulation portion.
FIG. 4 is a side view illustrating a state in which a segment included in a conventional endoscopic articulating tube is detached from an articulation portion due to an excessive bending operation.
FIG. 5 is a perspective view illustrating a state in which a C-shaped coupling portion and a C-shaped notch portion, which together serve as an articulation portion of each segment included in a conventional endoscopic articulating tube, are symmetrically formed on both sides of each segment.
FIG. 6 is a side view illustrating a bending angle during a bending operation of a segment included in a conventional endoscopic articulating tube.
FIG. 7 is a perspective view illustrating an example of an endoscope, a cable, and an operating unit to which the present disclosure is applied.
FIG. 8 is a perspective view illustrating an overall configuration of an endoscopic articulating tube according to the present disclosure.
FIG. 9 is a partially enlarged perspective view illustrating an articulation portion of an endoscopic articulating tube according to the present disclosure in more detail.
FIG. 10 is an exploded perspective view illustrating the configuration of segments included in an endoscopic articulating tube according to the present disclosure.
FIG. 11 is an exploded side view illustrating the detailed configuration and the coupling state of segments included in an endoscopic articulating tube according to the present disclosure.
a) and b) of FIG. 12 are side views illustrating a flexion and extension motion of adjacent segments and bending angles thereof according to the present disclosure.
a), b), and c) of FIG. 13 are side views illustrating angles of cape block portions and bay space portions located in respective first-first, first-second, and first-third coupling parts of a first coupling unit and second-first, second-second, and second-third coupling parts of a second coupling unit in each segment according to the present disclosure.

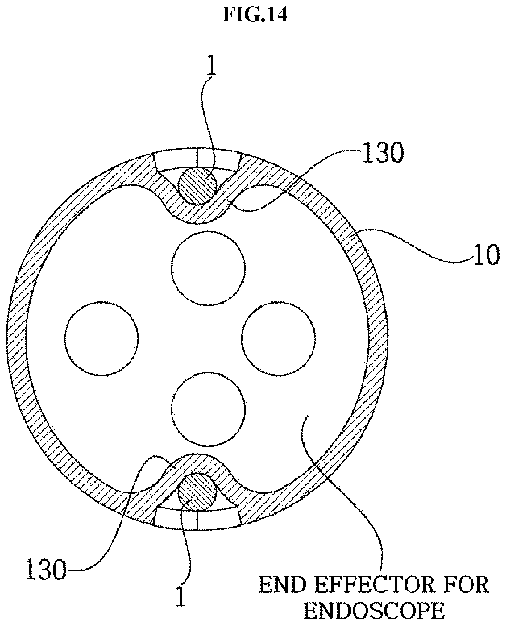

FIG. 14 is a cross-sectional front view illustrating an example in which a wire that causes a flexion and extension motion is inserted into a wire support portion of a segment included in an endoscopic articulating tube according to the present disclosure.

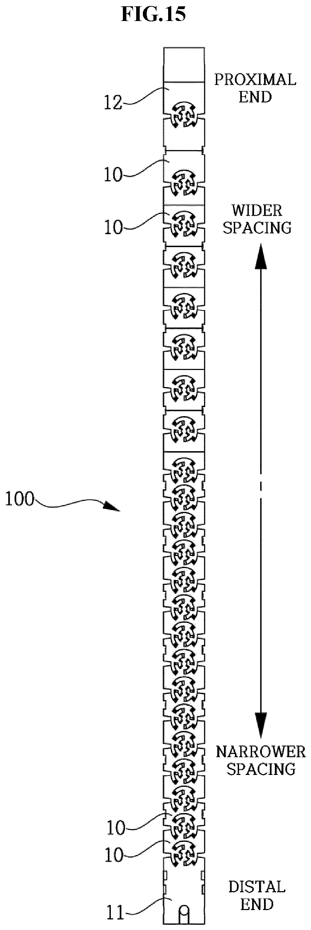

FIG. 15 is a side view illustrating an example in which segments included in an endoscopic articulating tube according to the present disclosure are arranged so that respective lengths of the segments gradually decreases from a proximal portion toward a distal portion.

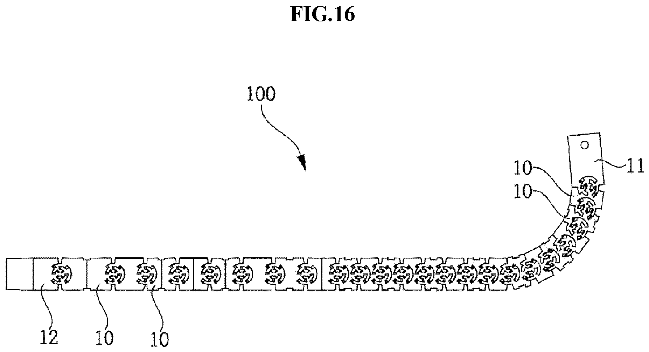

FIG. 16 is a side view illustrating a bending radius in a bent state by a flexion and extension motion of an endoscopic articulating tube according to the present disclosure.

### DETAILED DESCRIPTION

Hereinafter, embodiments disclosed in this specification will be described in detail with reference to the accompanying drawings, and regardless of the drawing reference numerals, identical or similar components are assigned the same reference numerals, and redundant descriptions thereof will be omitted. In addition, in describing the embodiments disclosed in this specification, if it is determined that a detailed description of a related known technology may obscure the gist of the embodiments disclosed in this specification, such detailed description will be omitted.

Terms including ordinal numbers such as first, second, and the like may be used to describe various components, but such components are not limited by the terms. The above terms are used solely to distinguish one component from another.

Singular expressions include plural expressions unless the context clearly indicates otherwise.

In this application, terms such as "include" or "have" are intended to specify the presence of a feature, number, step, operation, component, part or combination thereof described in the specification, but should be understood not to exclude in advance the possibility of the presence or addition of one or more other features, numbers, steps, operations, components, parts or combinations thereof.

Hereinafter, the present disclosure will be described in detail with reference to the accompanying drawings.

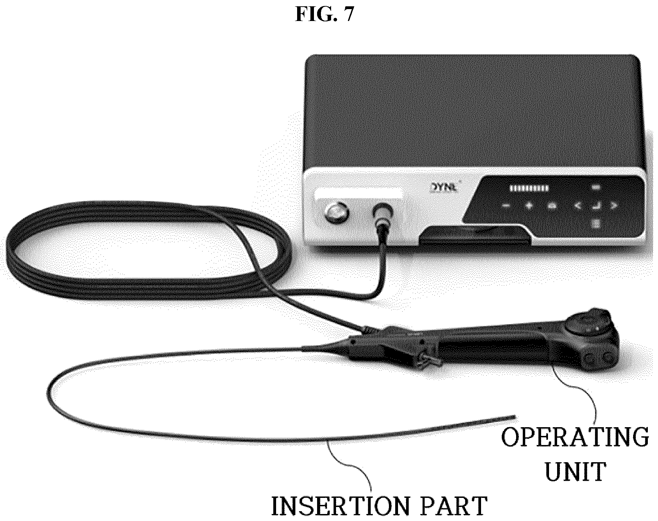

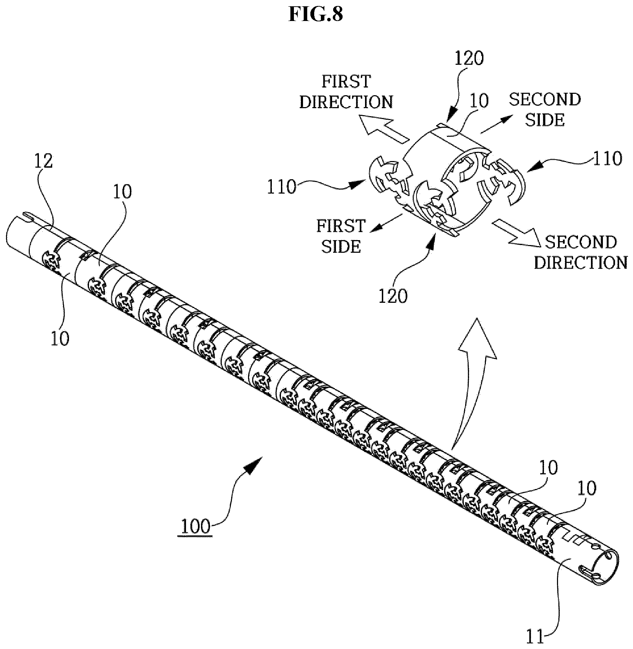

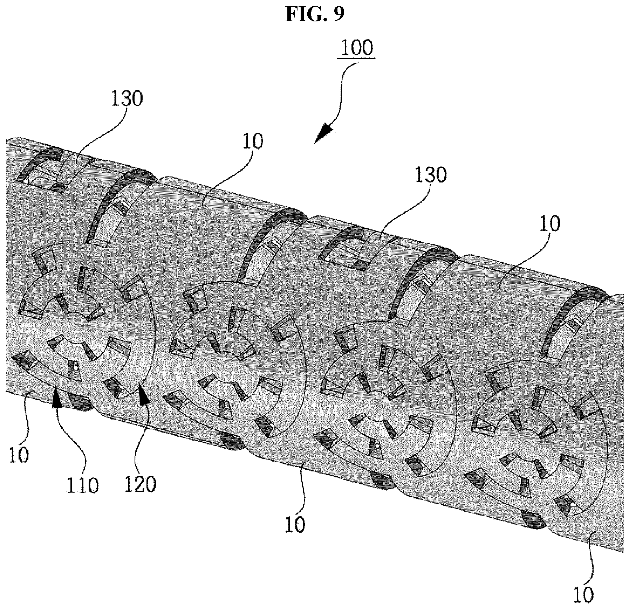

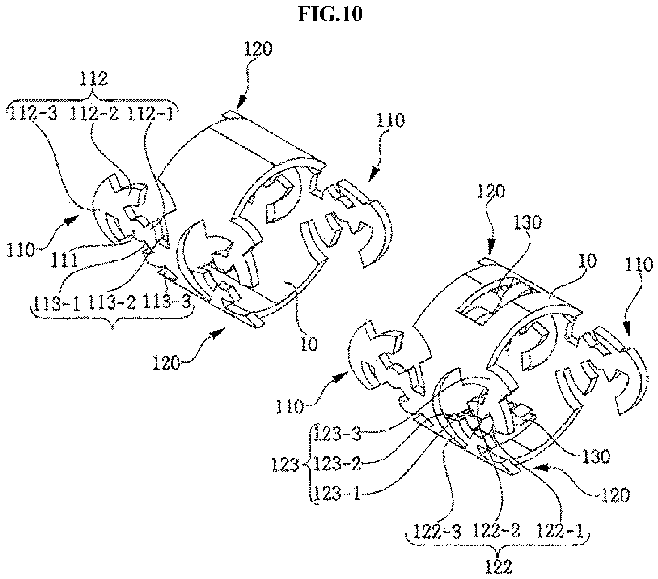

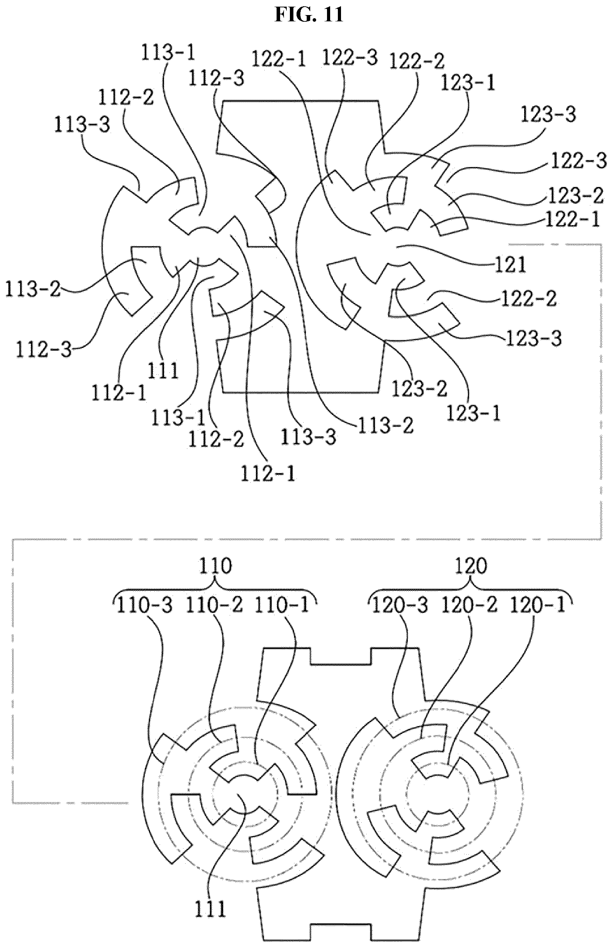

As illustrated in FIGS. 7 to 11, the present disclosure relates to an endoscopic articulating tube 100 including a plurality of tubular unit segments 10 that are articulately connected in series, allowing an endoscopic insertion unit to perform a flexion and extension motion by pulling and releasing a wire under control of a separate operating unit. The endoscope articulating tube 100 may include: a first coupling unit 10 including a plurality of coupling parts concentrically arranged around a spherical block portion 111 protruding from one side connecting end of each of the segments 10, each coupling part including a cape block portion 112 and a bay space portion 113 disposed at different radii and respectively forming an acute angle and an obtuse angle; and a second coupling unit 120 including a plurality of coupling parts concentrically arranged around a spherical space portion 121 provided at the other side connecting end of each of the segments 10, each coupling part including a bay space portion 122 and a cape block portion 123 disposed at different radii and respectively forming an obtuse angle and an acute angle. The spherical block portion 111, the cape block portion 112, and the bay space portion 113 of the first coupling unit 110 may be put-in and articulately coupled to the spherical space portion 121, the bay space portion 122, and the cape block portion 123 of the second coupling unit 120 of another segment 10. In this case, the term "put-in" refers to a state in which a protrusion is tightly inserted and fitted into a groove or the like to be properly engaged therewith.

In the present disclosure, the first coupling unit 110 and the second coupling unit 120 serve as an articulation portion configured to perform a flexion and extension motion while connecting respective segments 10 forming the endoscopic articulating tube 100, and the first coupling unit 110 and the second coupling unit 120 are formed by a staggered coupling structure of a plurality of coupling parts arranged at different radii on concentric circles, that is, by a plurality of put-in structures. Accordingly, detachment of the articulation portion during a flexion and extension motion in the course of an endoscopic examination, procedure, or surgery may be prevented.

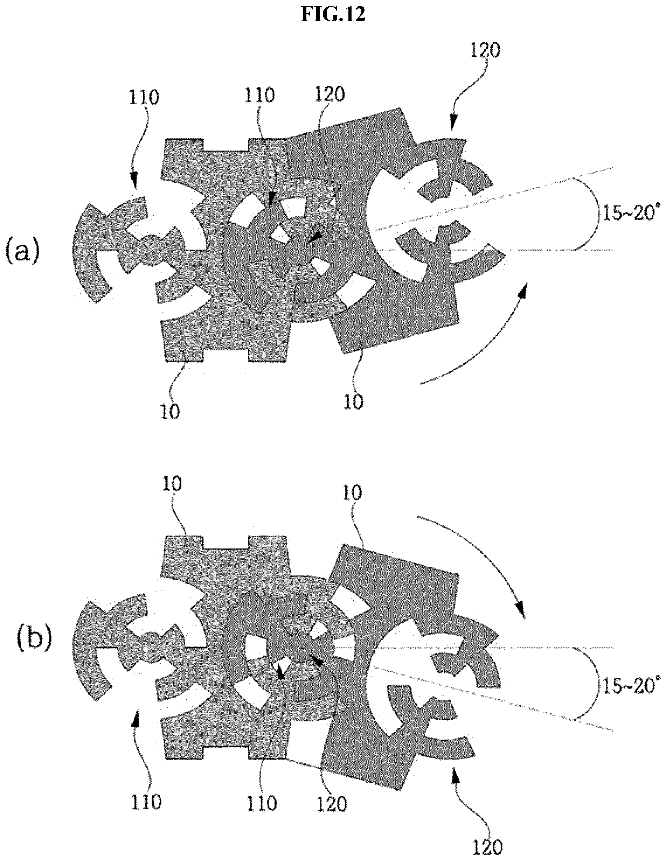

In addition, as illustrated in FIG. 12, the bay space portions 113 and 122 are each formed at an obtuse angle greater than 90 degrees (e.g., 110 degrees), and the cape block portions 112 and 123 are each formed at an acute angle less than 90 degrees (e.g., 70 degrees). Thus, a rotation angle (15 to 20 degrees) by which the cape block portions 112 and 123 rotate while sliding between the bay space portions 113 and 122 may be maximized. As a result, the bending radius according to a flexion and extension motion of the entire endoscopic articulating tube 100 may be minimized, thereby facilitating the flexion and extension motion in a state where the endoscopic articulating tube 100 is inserted into a narrow region of the human body.

Here, each segment 10 having the first and second coupling units 110 and 120 may be formed by cutting and perforating a tubular body having a long axis by laser irradiation.

In this way, according to the present disclosure, the tubular body prepared to match the long axis, i.e., a length of the endoscopic insertion unit, is irradiated with a laser to form incisions and perforations, thereby forming each segment 10 having the first and second coupling units 110 and 120. As a result, compared to manufacturing and assembling each individual segment 10 in which the first and second coupling units 110 and 120 are formed, productivity may be improved due to ease of manufacturing, and since exposure of the manufacturing and assembly processes to a contaminated environment is minimized, a hygienic endoscope articulating tube may be provided.

Meanwhile, in the present disclosure, the first coupling unit 110 may be provided on a first side in a first direction, which corresponds to one side connecting end of each of the segments 10, and the second coupling unit 120 may be provided on a second side in the first direction. In addition, the second coupling unit 120 may be provided on the first side in a second direction, which corresponds to the other side connecting end of each of the segments 10, and the first coupling unit 110 may be provided on the second side in the second direction.

To provide a better understanding of the present disclosure, the related art will be described again. As illustrated in FIG. 5, in the related art, a first C-shaped coupling portion 101 and a first C-shaped notch portion 102 are symmetrically formed on both facing sides of one end of each segment 10, and a second C-shaped coupling portion 111, a second C-shaped notch portion 112, and a spherical projection 113 are symmetrically formed on both facing sides of the other end. Therefore, in the medical field, a problem frequently occurs in which a put-in portion formed among the two coupling portions, the two notch portions, and the spherical projection became disengaged after deviating beyond an allowable rotation angle when a pair of left and right wires configured to be pulled or released by the operating unit for a flexion and extension motion of the articulating structure are forcibly pulled.

In contrast, the first coupling unit 110 is diagonally arranged on the first side in the first direction, which corresponds to one side connecting end of the segment 10, and on the second side in the second direction, which corresponds to the other side connecting end. Correspondingly, the second coupling unit 120 is diagonally arranged on the first side in the second direction, which corresponds to the other side connecting end, and on the second side in the first direction, which corresponds to one side connecting end. That is, the put-in direction of the first coupling unit 110 and the second coupling unit 120 on the first side are reversed and formed opposite to the put-in direction of the first coupling unit 110 and the second coupling unit 120 on the second side. Accordingly, even when a pair of left and right wires configured to be pulled or released by an operating unit are forcibly pulled, disengagement of a put-in portion due to deviation beyond an allowable rotation angle may be prevented.

At this time, the first coupling unit 110 may be formed of a first-first coupling part 110-1, a first-second coupling part 110-2, and a first-third coupling part 110-3 along three concentric circles having different radii centered around a spherical block portion 111.

For reference, a concentric circle refers to two or more circles with different radii and the same center, and in the present disclosure, the first-first coupling part 110-1, the first-second coupling part 110-2, and the first-third coupling part 110-3 are positioned on the concentric circles with different radii centered around the spherical block portion 111.

In this case, the first-first coupling part 110-1 may include a plurality of first-first cape block portions 112-1 and first-first bay space portions 113-1 alternately arranged around a first concentric circle surrounding the spherical block portion 111; the first-second coupling part 110-2 may include a plurality of first-second cape block portions 112-2 and first-second bay space portions 113-2 alternately arranged around a second concentric circle surrounding the first-first coupling part 110-1; and the first-third coupling part 110-3 may include a plurality of first-third cape block portions 112-3 and first-third bay space portions 113-3 alternately arranged around a third concentric circle surrounding the first-second coupling part 110-2.

The first-first cape block portions 112-1, the first-second bay space portions 113-2, and the first-third cape block portions 112-3 may be partially alternately formed in a radial direction of the concentric circles, and the first-first bay space portions 113-1, the first-second cape block portions 112-2, and the first-third bay space portions 113-3 may be partially alternately formed in the radial direction of the concentric circles.

In addition, the second coupling unit 120 may be formed of a second-first coupling part 120-1, a second-second coupling part 120-2, and a second-third coupling part 120-3 formed around concentric circles having three different radii centered around a spherical space portion 121.

In this case, the second-first coupling part 120-1 may include a plurality of second-first cape block portions 123-1 and second-first bay space portions 122-1 alternately arranged around a first concentric circle surrounding the spherical space portion 121; the second-second coupling part 120-2 may include a plurality of second-second cape block portions 123-2 and second-second bay space portions 122-2 alternately arranged around a second concentric circle surrounding the second-first coupling part 120-1; and the second-third coupling parts 120-3 may include a plurality of second-third cape block portions 123-3 and second-third bay space portions 122-3 alternately arranged around a third concentric circle surrounding the second-second coupling part 120-2.

In this case, the second-first cape block portions 123-1, the second-second bay space portions 122-2, and the second-third cape block portions 123-3 may be partially alternately formed in the radial direction of the concentric circles, and the second-first bay space portions 122-1, the second-second cape block portions 123-2, and the second-third bay space portions 122-3 may be partially alternately formed in the radial direction of the concentric circles.

In this configuration, when two adjacent segments 10 are connected in a straight line direction, the spherical block portion 111 of the first coupling unit 110 may be coupled to the spherical space portion 121 of the second coupling unit 120. In addition, each of the first-first, first-second, and first-third cape block portions 112-1, 112-2, and 112-3 and the first-first, first-second, and first-third bay space portions 113-1, 113-2, and 113-3, which form the first-first coupling part 110-1, the first-second coupling part 110-2, and the first-third coupling part 110-3 of the first coupling unit 110, may be coupled to a corresponding one of the second-first, second-second, and second-third cape block portions 123-1, 123-2, and 123-3 and the second-first, second-second, and second-third bay space portions 122-1, 122-2, and 122-3, which form the second-first coupling part 120-1, the second-second coupling part 120-2, and the second-third coupling part 120-3 of the second coupling unit 120. Accordingly, each cape block portion may be rotatably movable within a corresponding bay space portion within a predetermined angular range.

In this case, the first-first, first-second, and first-third cape block portions 112-1, 112-2, and 112-3, the first-first, first-second, and first-third bay space portions 113-1, 113-2, and 113-3, the second-first, second-second, and second-third bay space portions 122-1, 122-2, and 122-3, and the second-first, second-second, and second-third cape block portions 123-1, 123-2, and 123-3 may have angles as illustrated in FIG. 13.

In FIG. 13, a) illustrates an angle of a second-first cape block portion 123-1 configured to be put-in to a first-first bay space portion 113-1 of the first-first coupling part 110-1. The first-first bay space portion 113-1 is formed at an obtuse angle of 110 degrees, providing a slide path. The second-first cape block portion 123-1 is formed at an acute angle of 70 degrees and put-in to the slide path, thereby sliding in an arc in a forward or reverse direction depending on the rotation direction.

In FIG. 13, b) illustrates an angle of a second-second cape block portion 123-2 configured to be put-in to a first-second bay space portion 113-2 of the first-second coupling part 110-2. The first-second bay space portion 113-2 is formed at an obtuse angle of 110 degrees, providing a slide path. The second-second cape block portion 123-2 is formed at an acute angle of 70 degrees and put-in to the slide path to slide in an arc in a forward or reverse direction depending on a rotation direction.

In FIG. 13, c) illustrates an angle of a second-third cape block portion 123-3 configured to be put-in to a first-third bay space portion 113-3 of first-third coupling part 110-3. The first-third bay space portion 113-3 is formed at an obtuse angle of 110 degrees, providing a slide path. The second-third cape block portion 123-3 is formed at an acute angle of 70 degrees and put-in to the slide path to slide in an arc in a forward or reverse direction depending on a rotation direction.

In addition, the first and second coupling units 110 and 120 may be provided to protrude at one side connecting end and the other side connecting end of each segment 10. Flexion grooves may be provided on outer circumferential surfaces of the one connecting end and the other connecting end, excluding the areas where the first and second coupling units are provided to protrude, to form a block gap between segments and secure an articulation range during bending.

In this case, the flexion grooves may be formed naturally by the protrusion of the first and second coupling units 110 and 120. If necessary, the flexion grooves may also be intentionally formed with a curved shape to further secure the articulation range during bending. The size, angle, and shape of each flexion groove and of the first and second coupling units 110 and 120 may be pre-adjusted to determine a final bending angle between the segments 10, and such pre-adjustment may be preset and applied in advance according to a purpose of the endoscope or a target site of a treatment or surgical procedure.

In addition, as shown in FIG. 14, wire support portions 130, each formed as an inwardly recessed cutout, may be provided on upper and lower outer circumferential surfaces of each segment 10 in a direction orthogonal to the direction in which the first and second coupling units 110 and 120 are provided.

In this case, the wire support portions 130 may be provided in every other segment among the continuously connected segments 10, thereby being arranged in a stepping-stone pattern.

The wire support portion 130 may allow the wire 1 to be maintained in a stable supporting state at an accurate position when pulled by operation of the operating unit.

In addition, a segment 10 located at one end among the segments 10 included in the articulating tube 100 may be coupled to a separate effector segment 11 into which an end effector is inserted and fixed, and a segment 10 located at the other end among the segments 10 may be provided with a separate coupling segment 12 connected to an operating unit.

In this case, the effector segment 11 and the separate coupling segment 12 may be connected in the same structure as an articulating structure in which the segments 10 are connected to each other and perform a flexion and extension motion.

In addition, as illustrated in FIG. 15, the segments 10 continuously arranged in a straight direction to form the articulating tube 100 may be arranged such that a length of a tubular body gradually becomes shorter toward a distal end where the end effector is located.

In this configuration, when a flexion and extension motion occurs at each articulation portion of the segments 10 as the wire 1 is pulled by operation of the operating unit, bending spacing between segments 10 located in the direction of the distal end where the end effector is located becomes narrower, as shown in FIG. 16, thereby minimizing a bending radius.

In the present disclosure, as described above, the bay space portions respectively provided in the first coupling unit 110 and the second coupling unit 120 are each formed at an obtuse angle greater than 90 degrees so as to secure a maximum rotation angle, and the segments 10 toward the distal end where the end effector is located are formed to have shorter lengths so that bending spacing between the segments 10 becomes narrower. Accordingly, the bending radius of the entire endoscopic articulating tube 100 is minimized. In addition, by minimizing the bending radius in this manner, examination, treatment, or surgery may be performed easily and precisely when the tube is inserted into a narrow region of the human body.

The technical features disclosed in the various embodiments of the present disclosure are not necessarily limited to the respective embodiments, and unless mutually incompatible, such features may be combined with those of other embodiments.

Therefore, although each embodiment has been described with emphasis on its own specific technical features, such features may be used in combination with others, provided that they are not mutually incompatible.

The present disclosure is not limited to the embodiments and the accompanying drawings described above. Various modifications and variations may be made by those skilled in the art without departing from the scope of the disclosure. Accordingly, the scope of the present disclosure should be defined by the claims and their equivalents, including all modifications and variations that fall within the spirit and scope of the claims.

In the present disclosure, the first coupling unit and the second coupling unit serve as an articulation portion configured to perform a flexion and extension motion while connecting respective segments forming the endoscopic articulating tube, and the first coupling unit and the second coupling unit are formed by a staggered coupling structure of a plurality of coupling parts arranged at different radii on concentric circles, that is, by a plurality of put-in structures. Accordingly, it is possible to prevent detachment of the articulation portion during a flexion and extension motion in the course of an endoscopic examination, procedure, or surgery.

In the present disclosure, as the bay space portions are each formed at an obtuse angle greater than 90 degrees, and the cape block portions are each formed at an acute angle less than 90 degrees, a rotation angle by which the cape block portions rotate while sliding between the bay space portions may be maximized. Accordingly, it is possible to minimize a bending radius according to a flexion and extension motion of the entire endoscopic articulating tube, thereby facilitating the flexion and extension motion in a state where the endoscopic articulating tube is inserted into a narrow region of the human body.

In the present disclosure, since the put-in directions of the first and second coupling units on the first side and the put-in directions of the first and second coupling units on the second side are reversed and formed in opposite directions, it is possible to prevent disengagement of the put-in portion caused by a deviation beyond a rotation angle when a pair of left and right wires configured to be pulled or released by an operating unit is forcibly pulled.

In the present disclosure, the segments continuously arranged in a straight direction to form the articulating tube are arranged such that a length of a tubular body gradually becomes shorter toward a distal end where the end effector is located. Accordingly, when a flexion and extension motion occurs at the articulation portion of each segment as the wire is pulled by operation of the operating unit, bending spacing between segments located in the direction of the distal end becomes narrower, thereby minimizing a bending radius.

In the present disclosure, the bending radius may be minimized by forming the bay space portions at an obtuse angle to secure a maximum rotation angle and by forming the lengths of the segments in the direction of the distal end where the end effector is located to be shorter so as to narrow bending spacing between the segments. By minimizing the bending radius in this way, it is possible to conveniently and precisely perform examination, treatment, or surgery in a narrow region of the human body.

## Claims

1. An endoscopic articulating tube comprising a plurality of tubular unit segments that are articulately connected in series, allowing an endoscopic insertion unit to perform a flexion and extension motion by pulling and releasing a wire under control of a separate operating unit, the endoscopic articulating tube comprising:
a first coupling unit comprising a plurality of coupling parts concentrically arranged around a spherical block portion protruding from one side connecting end of each of the segments, each coupling part comprising a cape block portion and a bay space portion disposed at different radii and respectively forming an acute angle and an obtuse angle; and
a second coupling unit comprising a plurality of coupling parts concentrically arranged around a spherical space portion provided at the other side connecting end of each of the segments, each coupling part comprising a bay space portion and a cape block portion disposed at different radii and respectively forming an obtuse angle and an acute angle,
wherein the spherical block portion, the cape block portion, and the bay space portion of the first coupling unit are put-in and articulately coupled to the spherical space portion, the bay space portion, and the cape block portion of the second coupling unit of an adjacent segment.

2. The endoscopic articulating tube of claim 1, wherein:
the first coupling unit is provided on a first side in a first direction, which corresponds to one side connecting end of each of the segments, and the second coupling unit is provided on a second side in the first direction, and
the second coupling unit is provided on a first side in a second direction, which corresponds to the other side connecting end of each of the segments, and the first coupling unit is provided on a second side in the same second direction.

3. The endoscopic articulating tube of claim 1, wherein the first coupling unit is formed of a first-first coupling part, a first-second coupling part, and a first-third coupling part around three concentric circles having different radii centered around a spherical block portion.

4. The endoscopic articulating tube of claim 3, wherein:
the first-first coupling part comprises a plurality of first-first cape block portions and first-first bay space portions alternately arranged around a first concentric circle surrounding the spherical block portion, and
the first-second coupling part comprises a plurality of first-second cape block portions and first-second bay space portions alternately arranged around a second concentric circle surrounding the first-first coupling part, and
the first-third coupling part comprises a plurality of first-third cape block portions and first-third bay space portions alternately arranged around a third concentric circle surrounding the first-second coupling part.

5. The endoscopic articulating tube of claim 4, wherein:
the first-first cape block portion, the first-second bay space portion, and the first-third cape block portion are partially alternately formed in a radial direction of the concentric circles, and
the first-first bay space portion, the first-second cape block portion, and the first-third bay space portion are partially alternately formed in the radial direction of the concentric circles.

6. The endoscopic articulating tube of claim 1, wherein the second coupling unit s formed of a second-first coupling part, a second-second coupling part, and a second-third coupling part disposed around three concentric circles having different radii centered around a spherical space portion.

7. The endoscopic articulating tube of claim 6, wherein:
the second-first coupling part comprises a plurality of second-first cape block portions and second-first bay space portions alternately arranged around the first concentric circle surrounding the spherical space,
the second-second coupling part comprises a plurality of second-second cape block portions and second-second bay space portions alternately arranged around a second concentric circle surrounding the second-first coupling part, and
the second-third coupling part comprises a plurality of second-third cape block portions and second-third bay space portions alternately arranged around a third concentric circle surrounding the second-second coupling part.

8. The endoscopic articulating tube of claim 7, wherein:
the second-first cape block portions, the second-second bay space portions, and the second-third cape block portions are partially alternately formed in the radial direction of the concentric circles, and
the second-first bay space portions, the second-second cape block portions, and the second-third bay space portions are partially alternately formed in the radial direction of the concentric circles.

9. The endoscopic articulating tube of claim 4 or 7, wherein, when two adjacent segments are connected in a straight line direction, the spherical block portion of the first coupling unit is coupled to the spherical space portion of the second coupling unit, and each of the cape block portions and bay space portions forming the first-first coupling part, the first-second coupling part, and the first-third coupling part of the first coupling unit is coupled to a corresponding one of the cape block portions and bay space portions forming the second-first coupling part, the second-second coupling part, and the second-third coupling part of the second coupling unit, so that each of the cape block portions is rotatably movable within a corresponding one of the bay space portions within a predetermined angular range.

10. The endoscopic articulating tube of claim 1, wherein
the first and second coupling units are provided to protrude at the one side connecting end and the other side connecting end of each of the segments, and
flexion grooves are provided on outer circumferential surfaces of one side connecting end and the other side connecting end, excluding areas where the first and second coupling units are provided to protrude, to secure an articulation range during bending.

11. The endoscopic articulating tube of claim 1, wherein wire support portions, each formed as an inwardly recessed cutout, are provided on upper and lower outer circumferential surfaces of each of the segments in a direction orthogonal to a direction in which the first and second coupling units are provided.

12. The endoscopic articulating tube of claim 11, wherein the wire support portions are arranged in every other segment among continuously connected segments, thereby being arranged in a stepping-stone pattern.

13. The endoscopic articulating tube of claim 1, wherein the segments continuously arranged in a straight direction to form the articulating tube are arranged such that a length of a tubular body gradually becomes shorter toward a distal end where the end effector is located.
